# EUROPEAN PATENT APPLICATION

(11) **EP 4 309 719 A1**
(43) Date of publication of application: **24.01.2024**
(21) Application number: 22771347.6
(22) Date of filing: 14.03.2022
(51) Int. Cl.: A61M 25/00, A61M 25/088

(54) **GUIDE EXTENSION CATHETER**

(30) Priority: 16.03.2021 JP 2021042084
(71) Applicant: Nipro Corporation, Osaka-shi, Osaka 531-8510 (JP)
(72) Inventor: TANIKAWA, Masahiro, Osaka-shi, Osaka 531-8510 (JP); YOSHIDA, Tsuyoshi, Osaka-shi, Osaka 531-8510 (JP)
(74) Representative: Slingsby Partners LLP
(86) International application number: PCT/JP2022/011164
(87) International publication number: WO 2022/196601

(57) **Abstract**

A guide extension catheter 10 including a distal shaft 12 and a proximal shaft 14, wherein: a main body 16 of the distal shaft 12 is placed on a support base 30 of a three-point bending test jig where a distance between fulcrums 32, 32 is 15 mm; an indenter 34 that approaches the support base 30 at a speed of 10 mm/min in a middle between the fulcrums 32, 32 is pressed against the main body 16 from a lateral side to deform the main body 16 until kinking occurs; and at that time, a maximum load value acting on the main body 16 is 0.1 to 0.6 N. A distal end tip 18 that is more flexible than the main body 16 is provided to the distal shaft 12. The distal end tip 18 has a length dimension of 2.5 mm or greater, and includes a marker part mixed with a powder made of a radiopaque material.

## Description

### TECHNICAL FIELD

This invention relates to a guide extension catheter used, for example, in percutaneous coronary intervention (PCI) to deliver a therapeutic catheter to a more distal lesion site.

### BACKGROUND ART

Percutaneous coronary intervention using a therapeutic catheter has been conventionally performed as a less invasive treatment method than surgical treatment by opening the chest for stenosis, obstruction and the like of a coronary artery in the heart, for example. Percutaneous coronary intervention is a procedure in which a therapeutic catheter is guided by a guiding catheter to the lesion site of a coronary artery, such as a stenosis part, and the therapeutic catheter is delivered to the lesion site on the distal side of the guiding catheter to provide treatment.

Meanwhile, guiding catheters are required to have excellent torque transmission efficiency, kink resistance, and the like, because, for example, excellent pushability is required considering the passability of the somatic lumen to the entrance of coronary artery. Therefore, it is difficult to insert a guiding catheter into a narrow and complicatedly serpentine coronary artery. Thus, when the therapeutic catheter is attempt to be inserted into the coronary artery from the distal opening of the guiding catheter, which is placed so as to be hooked on the entrance of the coronary artery, the therapeutic catheter may be unable to follow the curve of the coronary artery, making it impossible to insert the therapeutic catheter to the vicinity of the distal lesion site.

To support delivery of a therapeutic catheter to a more distal lesion site, a guide extension catheter inserted into a coronary artery is proposed in International Publication No. WO 2018/030075 A1 (Patent Document 1). The guide extension catheter is inserted through a guiding catheter, and is inserted into the coronary artery from the distal end of the guiding catheter. The guide extension catheter is inserted into the coronary artery through the lumen of the guiding catheter, which is inserted into the coronary artery beforehand. Thus, the guide extension catheter has a smaller diameter than that of the guiding catheter. The guide extension catheter has better followability with respect to serpentine coronary arteries than the guiding catheter and can reach more distally in coronary arteries, allowing the therapeutic catheter to be delivered to more distal lesion sites.

### BACKGROUND ART DOCUMENT

### PATENT DOCUMENT

Patent Document 1: WO 2018/030075 A1

### SUMMARY OF THE INVENTION

### PROBLEM THE INVENTION ATTEMPTS TO SOLVE

However, there are limits to the extent to which the guide extension catheters of conventional construction can be inserted in coronary arteries, etc. In order to treat the distal lesion sites in coronary arteries, etc. with therapeutic catheters, guide extension catheters that can be inserted more distally in coronary arteries, etc. are sometimes required.

It is therefore one object of the present invention to provide a guide extension catheter of novel structure which can be inserted more distally in coronary arteries, etc.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a guide extension catheter comprising: a proximal shaft; and a distal shaft of tubular shape provided on a distal end side of the proximal shaft, wherein regarding a main body of the distal shaft, a maximum load value acting on the main body is at least 0.1 N and not greater than 0.6 N when the main body is placed on a support base of a test jig of a three-point bending test where a distance between fulcrums is set to 15 mm and the main body is deformed until kinking occurs by an indenter which moves closer to the support base at a relative moving speed of 10 mm/min being pressed against the main body from a lateral side at a center portion between the fulcrums of the support base, the distal shaft includes a distal end tip that is provided on the distal end side with respect to the main body and is more flexible than the main body, and a length dimension of the distal end tip is not smaller than 2.5 mm, and the distal end tip includes a marker part in which a powder comprising a radiopaque material is mixed.

According to the guide extension catheter structured following the present preferred embodiment, the main body of the distal shaft reliably obtains followability with respect to the curve of a blood vessel or the like through bending deformation, and avoids the reduction in pushability and kink resistance due to excessive flexibility. Therefore, the distal shaft can achieve excellent followability with respect to the shape of the coronary artery, etc., and the distal shaft can be inserted more distally into the coronary artery, etc.

Besides, the distal end tip that is more flexible than the main body is provided to the distal shaft while setting a certain degree of hardness to the main body of the distal shaft. With this configuration, when the distal shaft passes through a sharply curved portion of a coronary artery, for example, the distal end tip that easily follows the curved shape of the coronary artery or the like leads the main body, which makes it easier for the distal end of the main body to face the direction of extension of the coronary artery, etc., so that the main body is easily guided by the distal end tip in the direction of extension of the coronary artery, etc.

In order to effectively realize such a guiding action of the distal end tip on the main body, the distal end tip need to have a length dimension that can be deformed into a curved shape that follows the coronary artery, etc. Therefore, the length dimension of the distal end tip must be small enough to allow the tip to be deformed into a curved shape that follows the coronary artery, etc. Therefore, by setting the length dimension of the distal end tip to 2.5 mm or greater, curving deformation of the distal end tip effectively improves crossability of the distal shaft with respect to the coronary artery, etc.

Conventional guide extension catheters have a ring marker attached to the distal end portion to ensure the visibility of the distal end portion under the X-ray fluoroscopy. This may have an adverse effect on insertability by limiting a flexible region in the distal end portion due to the rigid ring marker. Thus, in the present preferred embodiment, the marker part to ensure visibility under X-ray fluoroscopy is made of a material mixed with a powder comprising a radiopaque material, which provides sufficient flexibility. By providing such a flexible marker part on the distal end tip, it is possible to prevent the substantial length of the distal end tip from being shortened by attachment of the hard marker while ensuring the visibility of the distal end portion of the distal shaft under X-ray fluoroscopy, thereby realizing excellent followability of the distal shaft with respect to a blood vessel or the like.

It is desirable that the length of the distal end tip be, for example, not greater than 10 mm, although it depends on the flexibility and the like based on the material of the distal end tip. The reason is that if the flexible distal end tip is too long, the distal end portion of the distal shaft constituted by the distal end tip is likely to be deformed, and the lumen of the distal shaft may be crushed and blocked, or resistance during insertion into the blood vessel may be increased.

A second preferred embodiment provides the guide extension catheter according to the first preferred embodiment, wherein on an outer circumferential surface of the distal shaft, a coating layer of hydrophilic polymer is provided from a distal end toward a proximal end of the distal shaft over a region having a length that does not reach the proximal end of the distal shaft.

According to the guide extension catheter structured following the present embodiment, when the distal shaft is inserted into a guiding catheter or a coronary artery, slipperiness of the outer circumferential surface of the distal shaft is improved, making it easy to insert. In addition, the coating layer is provided over a region having a length that does not reach the proximal end of the distal shaft that should remain in the guiding catheter. This will prevent the distal shaft from being difficult to be positioned with respect to the guiding catheter due to the coating layer, thereby avoiding troubles such as the distal shaft slipping out of the guiding catheter, for example.

A third preferred embodiment provides the guide extension catheter according to the first or second preferred embodiment, wherein an anti-slip part is provided on an outer circumferential surface of a proximal end portion of the distal shaft.

By providing the anti-slip part to the proximal end portion of the distal shaft, which should remain in the guiding catheter, the distal shaft becomes easy to be positioned with respect to the guiding catheter. This makes it possible to avoid troubles such as the entire distal shaft being accidentally exposed from the guiding catheter.

A fourth preferred embodiment provides the guide extension catheter according to any one of the first through third preferred embodiments, wherein when kinking occurs in the main body of the distal shaft by the main body being wound around a kink test jig having an outer circumferential surface of round tubular shape, an outer diameter dimension of the kink test jig is not greater than 3 mm.

According to the guide extension catheter structured following the present preferred embodiment, the main body of the distal shaft is resistant to kinking, making it difficult to cause reduction in pushability, insertion failure of the therapeutic catheter due to kinking. Furthermore, the main body of the distal shaft can be bent without kinking until it is bent with a relatively small radius of curvature, which provides excellent followability with respect to serpentine coronary arteries.

A fifth preferred embodiment provides the guide extension catheter according to any one of the first through fourth preferred embodiments, wherein regarding the main body of the distal shaft, a maximum load value is not smaller than 4.0 N, the maximum load value being measured when the main body is clamped radially between a support surface and a crushing jig while being subjected to a load of 0.5 N and then the crushing jig is further moved 0.5 mm in a direction of approach to the support surface.

According to the guide extension catheter structured following the present preferred embodiment, the main body of the flexible distal shaft achieves excellent bending flexibility, which is considered to contribute to the followability with respect to coronary arteries or the like, while the cross-sectional shape is stably maintained by reliably obtaining the resistance to crushing in the radial direction. This makes it possible to prevent the contact area with the coronary artery or the like from increasing due to the distal shaft deforming into an elliptical cross-sectional shape, thereby reducing the resistance during insertion, for example.

A sixth preferred embodiment provides the guide extension catheter according to any one of the first through fifth preferred embodiments, wherein regarding the distal end tip, a maximum load value is at least 0.5 N and not greater than 1.5 N, the maximum load value being measured when the distal shaft connected to the distal end tip is restrained by a holding jig at a location shifted to a proximal end side from a portion extending for 5 mm from a distal end of the distal shaft while a distal end face of the distal end tip is in contact with a pressing jig and then the holding jig is moved closer to the pressing jig at a speed of 10 mm/min to compress the distal end tip by 1 mm in a length direction.

According to the guide extension catheter structured following the present preferred embodiment, the distal end tip is made sufficiently flexible while maintaining the hardness (firmness) that is necessary for insertion into coronary arteries, etc., thereby more advantageously obtaining the followability of the guide extension catheter with respect to coronary arteries, etc.

### EFFECT OF THE INVENTION

According to the present invention, the guide extension catheter can be inserted more distally in coronary arteries, etc.

### BRIEF DESCRIPTION OF THE DRAWINGS

FIG. 1 is a side view showing a guide extension catheter according to a first practical embodiment of the present invention.
FIG. 2 is an enlarged cross sectional view showing a principal part of the guide extension catheter of FIG. 1.
FIG. 3 is a view showing a three-point bending test of a main body of a distal shaft constituting the guide extension catheter of FIG. 1.
FIG. 4 is a view showing a flexibility test of a distal end portion of the distal shaft constituting the guide extension catheter of FIG. 1.
FIG. 5 is a view showing a kink resistance test of the distal shaft constituting the guide extension catheter of FIG. 1.
FIG. 6 is a view showing a crush test of the distal shaft constituting the guide extension catheter of FIG. 1.
FIG. 7 is a side view showing a catheter assembly including the guide extension catheter of FIG. 1.
FIG. 8 is a view showing the catheter assembly of FIG. 7 in use.
FIG. 9 is a photograph showing a test apparatus used for the followability test of the guide extension catheter.
FIG. 10A is a photograph showing results of a followability test in which guide extension catheters of Examples 1 to 6 were inserted into a left anterior descending branch of the test apparatus shown in FIG. 9.
FIG. 10B is a photograph showing results of a followability test in which guide extension catheters of Comparative Examples 1 to 6 were inserted into the left anterior descending branch of the test apparatus shown in FIG. 9.
FIG. 11A is a photograph showing results of a followability test in which the guide extension catheters of Examples 1 to 6 were inserted into a left circumflex of the test apparatus shown in FIG. 9.
FIG. 11B is a photograph showing results of a followability test in which the guide extension catheters of Comparative Examples 1 to 6 were inserted into the left circumflex of the test apparatus shown in FIG. 9.
FIG. 12A is a photograph showing results of a followability test in which the guide extension catheters of Examples 1 to 6 were inserted into a right coronary artery R1 of the test apparatus shown in FIG. 9.
FIG. 12B is a photograph showing results of a followability test in which the guide extension catheters of Comparative Examples 1 to 6 were inserted into the right coronary artery R1 of the test apparatus shown in FIG. 9.
FIG. 13A is a photograph showing results of a followability test in which the guide extension catheters of Examples 1 to 6 were inserted into a right coronary artery R2 of the test apparatus shown in FIG. 9.
FIG. 13B is a photograph showing results of a followability test in which the guide extension catheters of Comparative Examples 1 to 6 were inserted into the right coronary artery R2 of the test apparatus shown in FIG. 9.
FIG. 14 is a graph showing results of the three-point bending test of the main body of the distal shaft.
FIG. 15 is a graph showing results of measuring a length of a distal end tip of the distal shaft.
FIG. 16 is a graph showing results of the flexibility test of the distal end portion of the distal shaft.
FIG. 17 is a graph showing results of the kink resistance test of the distal shaft.
FIG. 18 is a graph showing results of the crush test of the distal shaft.

### EMBODIMENTS FOR CARRYING OUT THE INVENTION

The practical embodiments of the present invention will be described below with reference to the drawings.

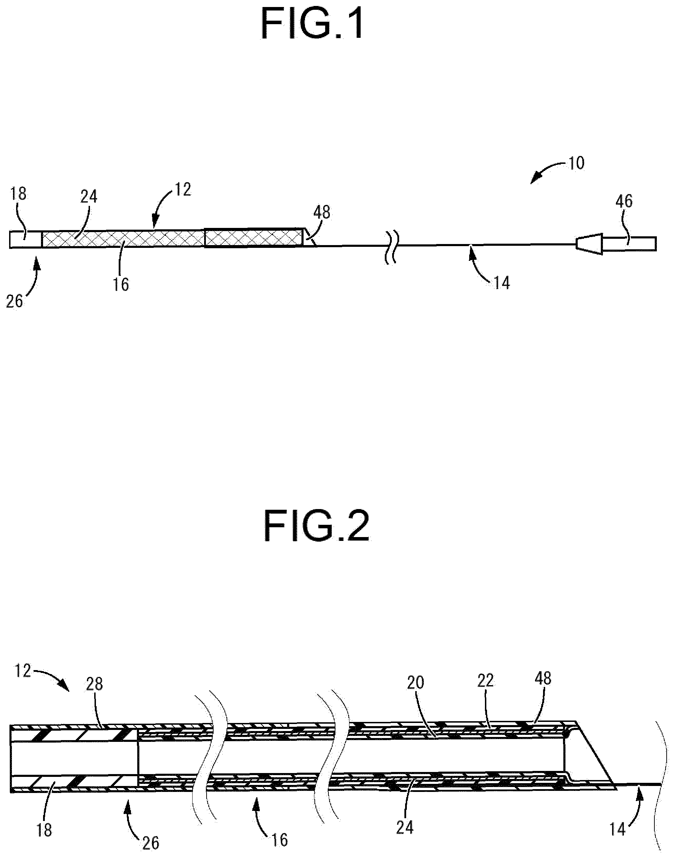

FIG. 1 shows a guide extension catheter 10 used for treatment of coronary arteries as a first practical embodiment of the invention. The guide extension catheter 10 is a rapid-exchange type catheter and includes a distal shaft 12 and a proximal shaft 14.

The distal shaft 12 is a tube having an approximately round tubular shape and is made of, for example, a soft synthetic resin or the like. The distal shaft 12 includes a main body 16 and a distal end tip 18 provided on the distal end side with respect to the main body 16.

The main body 16 of the distal shaft 12 is a so-called braid tube, which, as shown in FIG. 2, for example, has a structure in which a metal braided body 24 is arranged between an inner layer 20 and an outer layer 22 made of a synthetic resin or the like. The inner layer 20 and outer layer 22 are fastened by adhesion, welding or the like or integrally formed, for example, so that the braided body 24 is embedded therein. When the main body 16 has three layers of the inner layer 20, the outer layer 22, and the braided body 24, the inner layer 20 and the outer layer 22 may be made of mutually different materials or the same material. The inner layer 20 and the outer layer 22 are made of, for example, fluorine-based, polyamide-based, polyester-based, urethane-based or other synthetic resin. By having such a three-layer structure, the main body 16 has flexibility in the bending direction and improved load transmission efficiency in the axial direction, so that both followability with respect to the curve and pushability during insertion are realized.

The distal end tip 18, which constitutes a distal end portion 26 of the distal shaft 12, is more flexible than the main body 16 and easily deforms in response to load input. The distal end tip 18 can be made of a different material from that of the main body 16, but can also be made of the same material as that of the inner layer 20 and the outer layer 22 of the main body 16. The distal end tip 18 is made more flexible than the main body 16 due to, for example, the absence of the braided body 24 and the difference in the forming material.

The distal end tip 18 includes a marker part formed of, for example, a forming material made of a resin mixed with a powder comprising a radiopaque material. In this practical embodiment, the entirety of the distal end tip 18 is formed of a forming material mixed with a radiopaque powder, and the entirety of the distal end tip 18 serves as a marker part. However, it is not necessary that the entire distal end tip 18 be a marker part. For example, it would also be possible to provide a marker part partially on the distal end tip 18. For example, either the distal end portion or the proximal end portion of the distal end tip 18 can serve as a marker part, or a marker part can be provided in the middle of the distal end tip 18. The radiopaque powder used for the marker part is not limited in any particular way as long as the material has low X-ray transmissivity and has no significant adverse effect on the human body. For example, bismuth oxide, and metal powders such as tungsten which have higher visibility under the X-ray fluoroscopy are preferably adopted. The marker part is suitably made more flexible than the main body 16 of the distal shaft 12. However, for example, if the marker part is provided on a part of the distal end tip 18, the marker part can be made more rigid than the main body 16.

An axial length dimension of the distal end tip 18 is not smaller than 2.5 mm. Besides, it is desirable that the length of the distal end tip 18 be not greater than 10 mm. More preferably, the length of the distal end tip 18 is at least 3 mm and not greater than 7 mm, and in this practical embodiment, the length is about 4 mm.

The distal shaft 12 is provided with a coating layer 28. The coating layer 28 is formed of a hydrophilic polymer, and can be formed of, for example, polyvinylpyrrolidone (PVP), vinyl methyl ether-maleic anhydride copolymer (VEMA), acrylic or hyaluronic acid-based coating materials or the like. The coating layer 28 is provided so as to cover the outer circumferential surfaces of the distal end tip 18 and the main body 16 on the distal end side with respect to a connecting tube 48 described below. Besides, the coating layer 28 is provided on the outer circumferential surface of the distal shaft 12 from the distal end toward the proximal end of the distal shaft 12 over a region having a length that does not reach the proximal end of the distal shaft 12. The coating layer 28 is suitably provided over a region having a length that is half or more of the total length of the distal shaft 12, which in this practical embodiment is about 2/3 of the total length of the distal shaft 12. The coating layer 28 may be provided so as to cover approximately the entire outer circumferential surface of the distal shaft 12 from the distal end of the distal end tip 18 to the proximal end of the main body 16. In this case, for example, at the proximal end of the main body 16, the coating layer 28 is covered by the connecting tube 48 described below, so that the coating layer 28 is not exposed on the outer circumferential surface at the proximal end of the main body 16.

Regarding the main body 16 of the distal shaft 12, a maximum load value acting on the main body 16 when deformed until kinking occurs in a three-point bending test is not greater than 0.6 N. By setting the bending characteristics of the main body 16 as described above, the main body 16 is allowed to flex to the lateral side before kinking occurs, and has excellent flexibility to undergo lateral bending deformation even with respect to relatively small load inputs.

In addition, regarding the main body 16 of the distal shaft 12, a maximum load value acting on the main body 16 when deformed until kinking occurs in a three-point bending test is at least 0.1 N, more preferably at least 0.3 N. This allows the main body 16 of distal shaft 12 to have both flexibility and a certain degree of shape stability, so that when the guide extension catheter 10 is pushed toward the distal end side, the operating force is efficiently transmitted to the distal end side without unnecessary deformation of the main body 16.

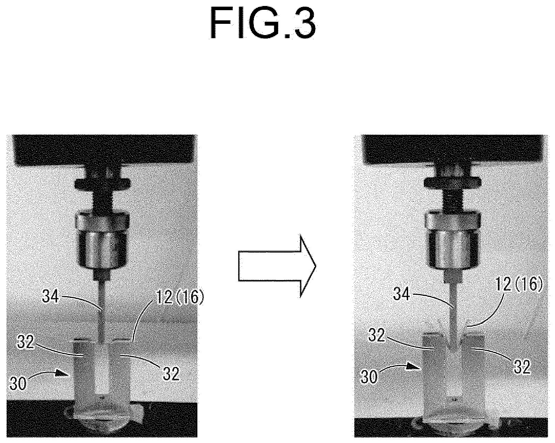

The three-point bending test can be performed as follows. As shown in FIG. 3, the person performing the test first places the main body 16 of the distal shaft 12 so that it straddles fulcrums 32, 32 on a support base 30 of the test jig, where the distance between the fulcrums 32, 32 is set to 15 mm. Next, an indenter 34, which moves closer to the support base 30 at a relative moving speed of 10 mm/min, is pressed against the main body 16 from the lateral side (from above in FIG. 3) to deform the main body 16 until kinking occurs. Then, the maximum load value acting on the main body 16 when kinking occurs is measured. The main body 16 of the distal shaft 12 used in the three-point bending test has a length of 50 mm.

It is desirable that the maximum load value acting on the main body 16 of the distal shaft 12 measured by the above three-point bending test be larger than the maximum load value acting on the distal end tip 18 measured by the same three-point bending test. Thus, the main body 16 of the distal shaft 12 has higher bending rigidity than the distal end tip 18, and when the operating force to push the guide extension catheter 10 toward the distal end side acts, a decrease in transmission efficiency of the operating force due to excessive deformation of the main body 16 is prevented. Furthermore, during abutment of the distal end tip 18, the distal end tip 18, which is more flexible than the main body 16 of the distal shaft 12, is deformed first in preference to the main body 16, thereby making it easier for the distal end tip 18 to face the direction of extension of a coronary artery 66 (described later).

Regarding the distal shaft 12, a maximum load value measured by a flexibility test of the distal end portion 26 is at least 0.5 N and not greater than 1.5 N. By setting the flexibility of the distal end portion 26 as described above, the distal end portion 26 can be flexibly deformed while maintaining a certain degree of shape stability against abutment in the axial direction.

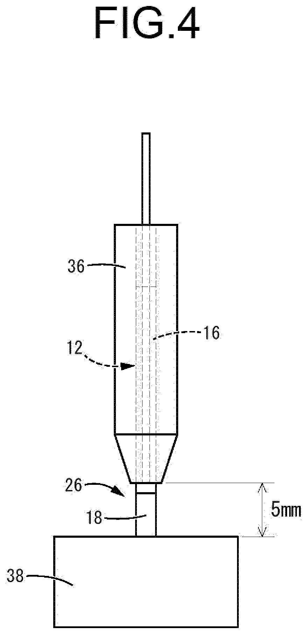

The flexibility test of the distal end portion 26 can be performed as follows. As shown in FIG. 4, the person performing the test first restrains the distal shaft 12 by a holding jig 36 at the location shifted to the proximal end side from a portion extending for 5 mm from the distal end of the distal shaft 12 to prevent deformation. Then, the distal end face of the distal end tip 18, which is the distal end face of the distal shaft 12, is overlapped with a pressing jig 38 in a state of contact. The holding jig 36 is constituted by, for example, combining a tubular outer tube part that restrains the outer circumferential surface of the distal shaft 12 and a columnar inner shaft part that restrains the inner circumferential surface of the distal shaft 12. Next, the holding jig 36 is moved closer to the pressing jig 38 at a speed of 10 mm/min to compress the distal end portion of the distal shaft 12 (the distal end tip 18) exposed from the holding jig 36 by 1 mm in the length direction, and the maximum load value at that time is measured.

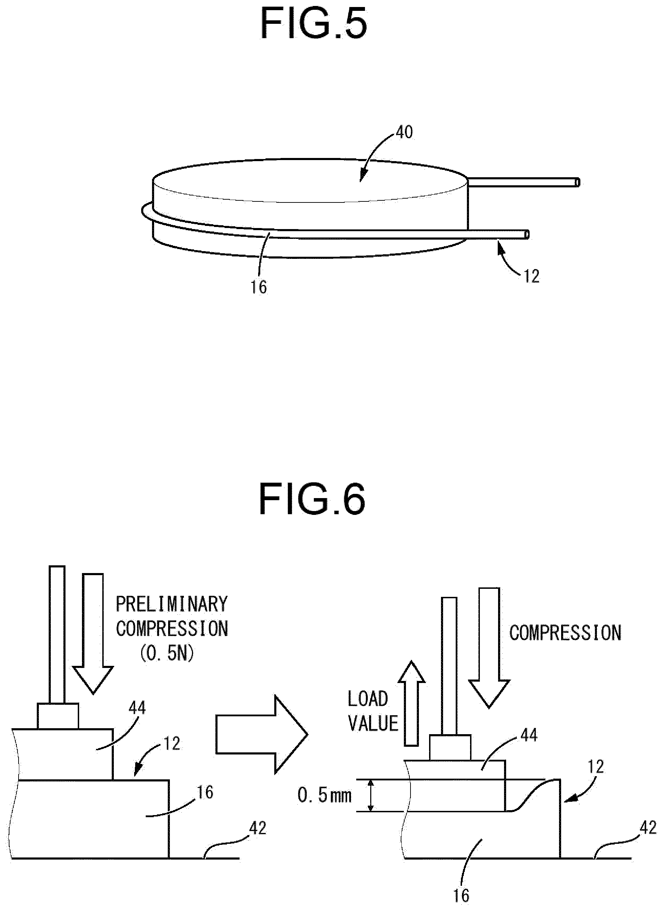

The main body 16 of the distal shaft 12 can kink when the main body 16 is curved with a small radius of curvature that is not greater than 3 mm in a kink resistance test by being wound around a kink test jig 40 having the outer circumferential surface of round tubular shape shown in FIG. 5. In other words, the main body 16 has excellent kink resistance such that kinking is difficult to occur due to bending deformation with a radius of curvature that is greater than 3 mm. By setting the kink resistance of the main body 16 as described above, the main body 16 is allowed to undergo bending deformation with a small radius of curvature without kinking, and poor force transmission due to kinking is unlikely to occur.

In the kink resistance test, the kink test jigs 40 equipped with the outer circumferential surfaces of different outer diameter dimensions are prepared. The main body 16 is subjected to curving deformation along the outer circumferential surface of the kink test jig 40 to check whether kinking occurs in the main body 16. Then, the person performing the test reduces the outer diameter dimension of the kink test jig 40 along which the main body 16 is placed in turn, and the outer diameter dimension of the kink test jig 40 when kinking occurs in the main body 16 is used as the test result. Therefore, the smaller the outer diameter dimension of the kink test jig 40, which is the test result of the kink resistance test, the more difficult it is for the main body 16 to kink due to bending deformation, and the better the kink resistance of the main body 16.

Regarding the main body 16 of the distal shaft 12, it is desirable that a maximum load value measured in a crush test be not greater than 6.0 N. By setting the crushing characteristics of the main body 16 as described above, the main body 16 is relatively easy to undergo crushing deformation (change in cross-sectional shape) in response to a lateral load, and is easy to undergo bending deformation with a change in cross-sectional shape, thereby realizing excellent bending flexibility.

Besides, regarding the main body 16 of the distal shaft 12, it is desirable that a maximum load value measured in the crush test be not smaller than 4.0 N. This prevents the cross-sectional shape of the main body 16 from changing more than necessary in response to input and makes it easier to maintain the lumen of the distal shaft 12. For example, by setting the maximum load value measured in the crush test of the main body 16 to be at least 4.0 N and not greater than 6.0 N, the excellent flexibility of the main body 16 against bending and the maintenance of the lumen of the main body 16 can be achieved in a compatible manner.

The crush test can be performed as follows. First, as shown in FIG. 6, the person performing the test clamps the main body 16 of the distal shaft 12 radially between a support surface 42 and a crushing jig 44 and applies a load of 0.5 N. Next, the crushing jig 44 is moved 0.5 mm in the direction of approach to the support surface 42, and the maximum load value is measured when the main body 16 is crushed further in the radial direction.

The proximal shaft 14 comprises a metallic coil wire (wire) such as medical stainless steel. On the proximal end side of the proximal shaft 14, a plate-shaped protecting member 46 is provided to prevent contact of a practitioner or the like with the proximal end of the proximal shaft 14. The outer circumferential surface of the proximal shaft 14 can be coated to improve slipperiness, corrosion resistance, and the like.

The distal end portion of the proximal shaft 14 is overlapped on the outer circumferential surface of the main body 16 of the distal shaft 12, and is connected to the main body 16 by the connecting tube 48 which is fastened externally about the main body 16. The connecting tube 48 is made of synthetic resin or the like, and is subjected to contracting deformation by means of heating or the like, for example, in a state of externally placed about the proximal end portion of the main body 16 on which the distal end portion of the proximal shaft 14 is overlapped. By so doing, the connecting tube 48 is fixed in close contact with the proximal end portion of the main body 16 of the distal shaft 12 and the distal end portion of the proximal shaft 14. By providing the connecting tube 48 in this manner, the proximal end portion of the main body 16 of the distal shaft 12 and the distal end portion of the proximal shaft 14 are fixed to each other by the connecting tube 48, and the distal end portion of the proximal shaft 14 is connected to the proximal end portion of the distal shaft 12. In this practical embodiment, the outer layer 22 and the braided body 24 of the main body 16 do not reach the proximal end of the distal shaft 12, and the proximal end of the distal shaft 12 is constituted by the inner layer 20 and connecting tube 48. However, at least one of the outer layer 22 and the braided body 24 may reach the proximal end of the distal shaft 12, and the inner layer 20 need not reach the proximal end of the distal shaft 12.

The aforementioned connection structure of the proximal shaft 14 and the distal shaft 12 is only an example and is not limited in particular. Specifically, for example, the distal end portion of the proximal shaft 14 may be fixed to the distal shaft 12 by being arranged and fastened between the inner layer 20 and the outer layer 22 over a predetermined length on the proximal end side of the distal shaft 12. In this case, the connecting tube 48 may be omitted. When the proximal shaft 14 is fastened between the inner layer 20 and the outer layer 22, it is desirable that the inner layer 20 and the outer layer 22 constitute the proximal end of the distal shaft 12, and the braided body 24 need not reach the proximal end of the distal shaft 12.

Furthermore, the connecting tube 48 is arranged at the proximal end portion of the distal shaft 12, so that the proximal end portion of the distal shaft 12 is provided with an anti-slip part without the coating layer 28 on the outer circumferential surface by means of the connecting tube 48. The coating layer 28 provided on the distal end side with respect to the connecting tube 48 is arranged on the outer circumferential surface of the distal shaft 12 from the distal end toward the proximal end of the distal shaft 12 over a region having a length that does not reach the proximal end of the distal shaft 12. The anti-slip part has a larger friction coefficient in blood against the vessel wall and a guiding catheter 50, etc., described later, than the portion having a small friction coefficient in blood against them due to the coating layer 28. The anti-slip part (the connecting tube 48) provided on the outer peripheral surface of the proximal end portion of the distal shaft 12 prevents the guide extension catheter 10 from slipping out of the guiding catheter 50 (described later) to the distal end side.

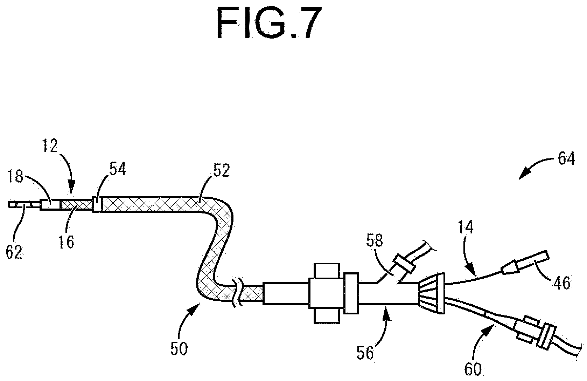

As shown in FIG. 7, the guide extension catheter 10 of the above construction is used by being inserted into the guiding catheter 50. Any conventional catheter known in the art can be suitably adopted as the guiding catheter 50. The guiding catheter 50 includes a bendable tubular catheter main body 52. The catheter main body 52 is a tube made of synthetic resin with a metallic reinforcement (a braided body) embedded therein, similar to the main body 16 of the guide extension catheter 10. At the distal end of the catheter main body 52, there is provided an imaging marker 54, which is made radiopaque by mixing a contrast agent or other means.

A Y connector 56 is provided at the proximal end side of the catheter main body 52. A check valve (not shown) is provided in the Y connector 56 to prevent backflow of blood. The Y connector 56 includes a side arm 58 that branches off from the main body, allowing injection of drug solutions, contrast media, etc. through the side arm 58.

A balloon catheter 60 serving as a therapeutic catheter is inserted into the guide extension catheter 10. For the balloon catheter 60, any conventionally known structure can be adopted. With the balloon catheter 60 inserted into a stenosis part 68 of the coronary artery 66, which is described later, a balloon 62 provided to the distal end portion of the balloon catheter 60 is inflated, whereby the stenosis part 68 is pushed to expand by the balloon 62. The therapeutic catheter is not limited to the balloon catheter 60 that pushes to expand the stenosis part 68 by balloon 62. As a specific example, it would be possible to adopt various known types of therapeutic catheters such as a cutting balloon catheter including a cutting balloon with a blade on its outer circumferential surface, a stent delivery catheter to indwell a stent in the stenosis part 68, an atherectomy catheter and a rotablator to remove the stenosis by cutting away the stenotic lesion.

A catheter assembly 64 is constituted by the guiding catheter 50, the guide extension catheter 10, and the balloon catheter 60. In the catheter assembly 64, the guide extension catheter 10 is inserted into the guiding catheter 50, and the balloon catheter 60 is inserted into the guide extension catheter 10.

Such a catheter assembly 64 is used, for example, to dilate the stenosis part 68 in the coronary artery 66 of the heart by percutaneous transluminal coronary angioplasty (PTCA). The following is a brief description of an example of the use of the catheter assembly 64 in a transfemoral approach procedure for reference.

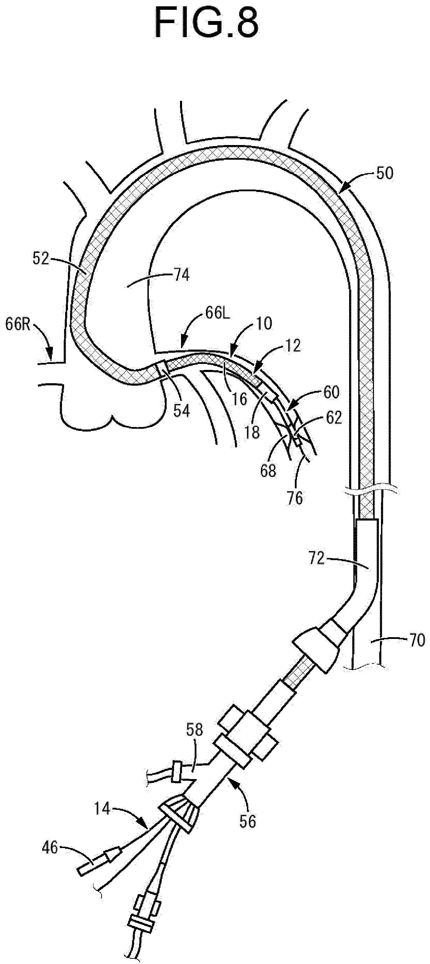

First, the practitioner punctures a femoral artery 70 of a patient with a needle (not shown), and inserts a sheath 72 from the puncture site into the femoral artery 70, as shown in FIG. 8. The practitioner inserts the guiding catheter 50, which is inserted into the femoral artery 70 through the sheath 72, into an ascending aorta 74, and places the distal end of the guiding catheter 50 at the entrance of the coronary artery 66.

Next, the practitioner places the guide extension catheter 10 externally about the guide wire 76 inserted through the guiding catheter 50, and the distal end portion of the distal shaft 12 of the guide extension catheter 10, which is pushed along the guide wire 76 within the guiding catheter 50, is protruded from the distal end of the guiding catheter 50. By so doing, the distal shaft 12 is inserted into the coronary artery 66, and the distal end of the distal shaft 12 is positioned in front of the stenosis part 68 in the coronary artery 66.

The distal end tip 18, which constitutes the distal end of the distal shaft 12, is made of resin mixed with a powder comprising a radiopaque material such as, for example, bismuth oxide and tungsten as a contrast medium, and the entire distal end tip 18 serves as a marker part that can be confirmed under the X-ray fluoroscopy. This allows the practitioner to deliver the guide extension catheter 10, which is inserted into the coronary artery, to the lesion site, by, for example, operating the guide extension catheter 10 while checking the position of the distal end tip 18 on a monitor displaying X-ray imaging. In particular, the flexible marker part makes it possible to use the distal end of the distal end tip 18 as a marker part, which enables fluoroscopy of the distal end of the distal shaft 12, which is not possible with a ring marker.

Subsequently, the practitioner inserts the balloon catheter 60, which is placed externally about the guide wire 76, through the distal shaft 12 of the guide extension catheter 10. The balloon 62 provided to the balloon catheter 60 is then protruded to the distal side of the distal shaft 12 and delivered to the stenosis part 68. The balloon 62 inserted into the stenosis part 68 is inflated inside of the stenosis part 68, and the stenosis part 68 is pushed to expand by the balloon 62, so that the blood flow in the coronary artery 66 is restored. The guide wire 76 used for insertion of the balloon catheter 60 may be different from the guide wire used for insertion of the guide extension catheter 10. For example, it would also be possible that, after the guide extension catheter 10 has been inserted through the guiding catheter 50, the guide wire is replaced by the thinner guide wire 76, and the balloon catheter 60 is inserted through the guide extension catheter 10.

The proximal end portion of distal shaft 12, which is located within the guiding catheter 50, includes the anti-slip part comprising the connecting tube 48 on its outer circumferential surface. This increases the frictional resistance acting between the proximal end portion of the guide extension catheter 10 and the guiding catheter 50, and the guide extension catheter 10 is positioned with respect to the guiding catheter 50. Therefore, for example, the guide extension catheter 10 can be prevented from slipping out of the guiding catheter 50 to the distal end side. Besides, for example, when the balloon catheter 60 protruding from the guide extension catheter 10 to the distal end side comes into contact with a stenotic lesion in a blood vessel and a force toward the proximal end side is exerted, it is possible to prevent the guide extension catheter 10 from being pushed back into the guiding catheter 50 by the contact reaction force of the balloon catheter 60. The connecting tube 48, which constitutes the anti-slip part, does not necessarily have to be located entirely within the guiding catheter 50, and for example, the distal end part may protrude distally from the guiding catheter 50.

The above is an example of using the catheter assembly 64 for a transfemoral approach, but it would also be possible, for example, to use the catheter assembly 64 for a transradial approach or a transbrachial approach. Furthermore, although FIG. 8 exemplified a procedure for the stenosis part 68 of the anterior descending branch of the left coronary artery 66L, the catheter assembly 64 can also be used, for example, for a procedure for the circumflex of the left coronary artery 66L or the right coronary artery 66R lesion. Besides, the use of the guide extension catheter 10, including the exemplified steps of the procedures (such as the order of insertion and removal of each catheter and guide wire) is only an example and is not limited in particular. In FIG. 8, the vasculature is shown schematically for illustrative purposes, with the descending aorta shown in a position that does not overlap with the coronary artery 66.

The guide extension catheter 10 protrudes from the distal end of the guiding catheter 50, which is placed at the entrance of the coronary artery 66, and is inserted into the coronary artery 66 until just before the stenosis part 68, thereby guiding a therapeutic catheter such as a balloon catheter 60 to the stenosis part 68. Hence, to make it possible to treat the stenosis part 68 even when it is far from the entrance of the coronary artery 66, the guide extension catheter 10 that can reach more distally in the coronary artery 66 is needed. Therefore, the guide extension catheter 10 structured following the present invention can be inserted more distally in the coronary artery 66 than the guide extension catheter of conventional structure.

Specifically, regarding the guide extension catheter 10, a maximum load value measured by the three-point bending test described above is at least 0.1 N and not greater than 0.6 N, and the distal end tip 18 has a length of 2.5 mm or greater. Besides, since the distal end tip 18 includes a flexible marker part mixed with a radiopaque powder, there is no rigid ring-shaped marker. These features allow the guide extension catheter 10 to be inserted more distally in the coronary artery 66 and to provide excellent crossability.

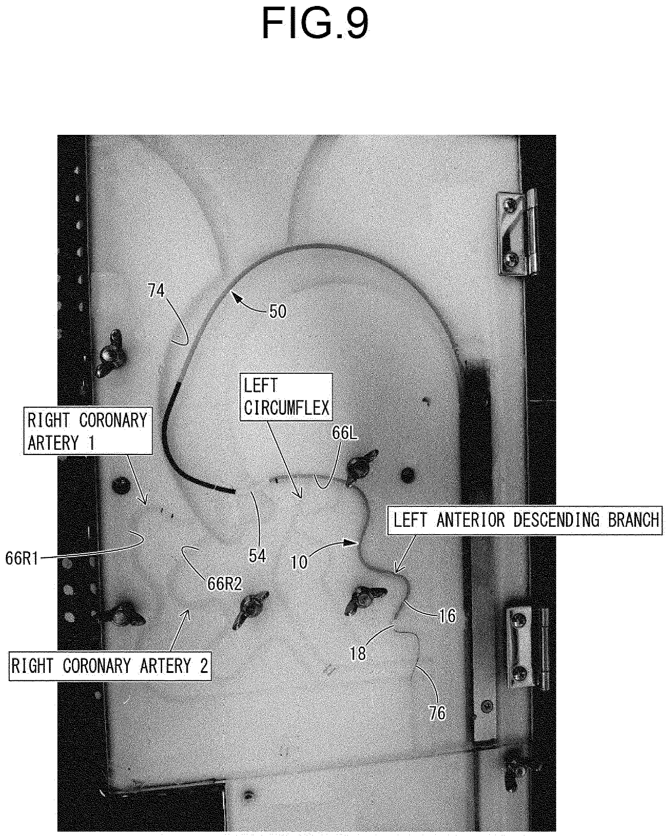

This is evident from the results of the followability test of the guide extension catheter with respect to a simulated coronary artery 66. Specifically, as shown in FIG. 9, a test apparatus with a cavity corresponding to a blood vessel such as a coronary artery 66 formed inside was prepared, and a guide extension catheter was inserted into the cavity corresponding to the coronary artery 66 at a speed of 500 mm/min to confirm followability (passability) of the guide extension catheter with respect to the coronary artery 66.

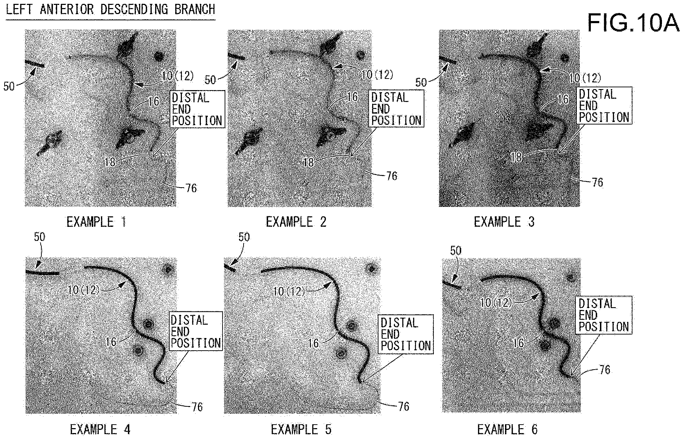

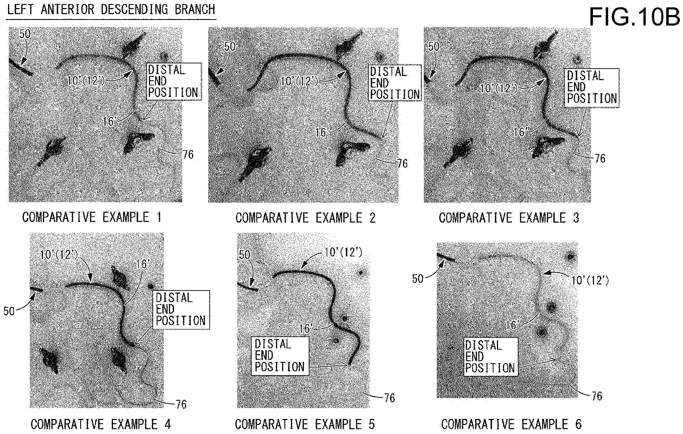

In the following description, Examples 1 to 6 are guide extension catheters according to the present invention, Comparative Examples 1 to 5 are guide extension catheters of conventional structure, and Comparative Example 6 is a guide extension catheter of Example 3 with a rigid ring-shaped imaging marker (a ring marker) attached so as to shorten the substantial length of the distal end tip. Besides, in FIGS. 10 to 13 below, the guide extension catheters of Examples 1 to 6 are assigned corresponding symbols to the guide extension catheter 10 of the preceding practical embodiment, and the guide extension catheters of Comparative Examples 1 to 6 are assigned symbols of a guide extension catheter 10', a distal shaft 12', and a main body 16'. The Examples 1 to 6 are all guide extension catheters 10 according to the present invention, and the forming materials, the structures, and the like are different from one another, for example, in the distribution of bending hardness of the main body 16 in the length direction and the like.

FIGS.10A and 10B show the results of the followability test of the guide extension catheter with respect to the left anterior descending branch of the left coronary artery 66L. The distal end positions of the guide extension catheters 10 of Examples 1 to 6, indicated by arrows in FIGS. 10A, all reached more distally than any of the guide extension catheters 10' of Comparative Examples 1 to 4, indicated by arrows in FIGS. 10B. Besides, the distal ends of the guide extension catheters 10' of Comparative Examples 5 and 6 reached approximately the same position as the distal ends of the guide extension catheters 10 of Examples 1 to 3 in the followability test with respect to the left anterior descending branch of the left coronary artery 66L, which is superior to the other Comparative Examples 1 to 4. However, Examples 4 to 6 showed excellent followability that can reach even more distally than Comparative Examples 5 and 6. Thus, it was demonstrated also by the tests that the guide extension catheter 10 according to the present invention can reach more distally in insertion into the left anterior descending branch of the left coronary artery 66L.

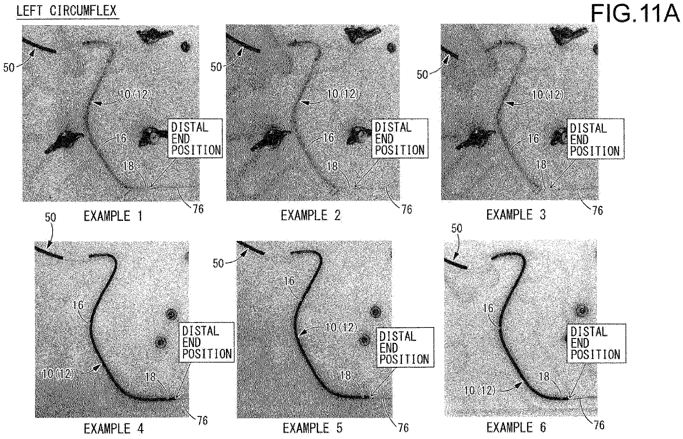

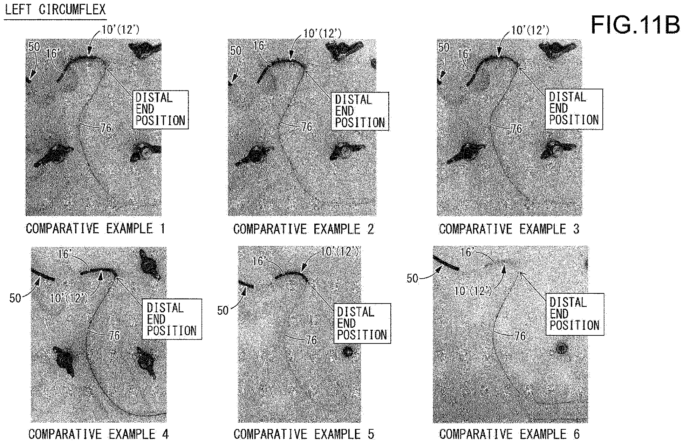

FIGS. 11A and 11B show the results of the followability test of the guide extension catheter to the left circumflex of the left coronary artery 66L. The distal end positions of the guide extension catheters 10 of Examples 1 to 6, indicated by arrows in FIG. 11A, all reached more distally than any of the guide extension catheters 10' of Comparative Examples 1 to 6, indicated by arrows in FIG. 11B. Thus, it was demonstrated also by the tests that the guide extension catheters 10 of Examples according to the present invention show extremely excellent followability, and can reach more distally compared to Comparative Examples in insertion into the left circumflex of the left coronary artery 66L.

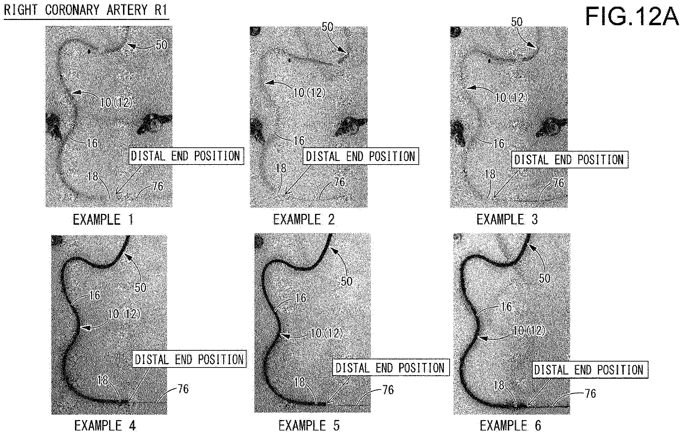

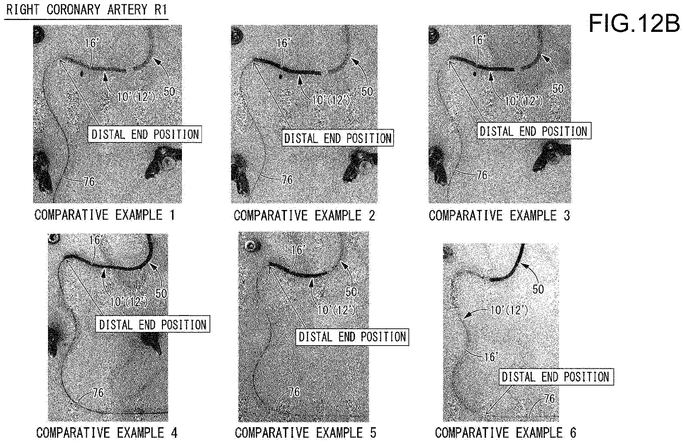

FIGS. 12A and 12B show the results of the followability test of the guide extension catheter with respect to the right coronary artery 66R1. The distal end positions of the guide extension catheters 10 of Examples 1 to 6, indicated by arrows in FIG. 12A, all reached significantly more distally than any of the guide extension catheters 10' of Comparative Examples 1 to 5, indicated by arrows in FIG. 12B. Besides, the distal end of the guide extension catheter 10' in Comparative Example 6 reached approximately the same position as the distal ends of the guide extension catheters 10 in Examples 2 and 3 in the followability test with respect to the right coronary artery 66R1, and showed better followability than the other Comparative Examples 1 to 5. However, Examples 1, and 4 to 6 showed excellent followability that can reach even more distally than Comparative Example 6. Thus, it was demonstrated also by the tests that the guide extension catheter 10 according to the present invention can reach more distally in insertion into the right coronary artery 66R1. Note that the proximal part of the right coronary artery 66R1 is raised compared to that of the right coronary artery 66R2, which will be described later.

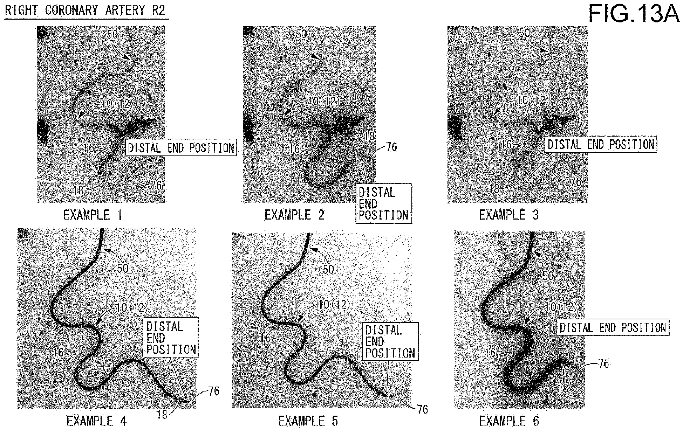

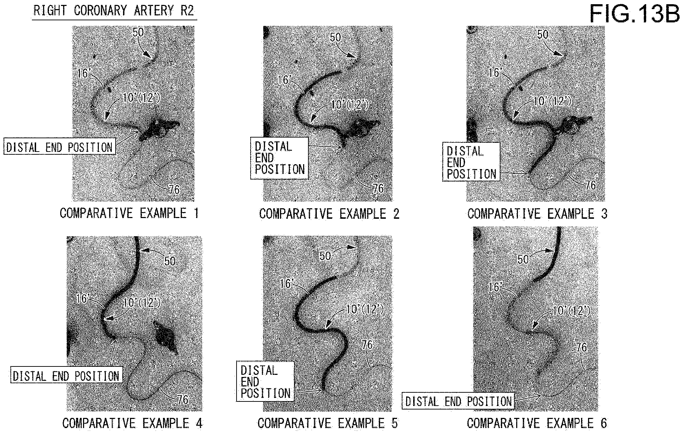

FIGS. 13A and 13B show the results of the followability test of the guide extension catheter with respect to the right coronary artery 66R2. The distal end positions of the guide extension catheters 10 of Examples 1 to 6, indicated by arrows in FIG. 13A, all reach more distally than any of the guide extension catheters 10' of Comparative Examples 1 to 5, indicated by arrows in FIGS. 13B. Thus, it was demonstrated also by the tests that the guide extension catheter 10 according to the present invention can reach more distally in insertion into the right coronary artery 66R2. The distal end of the guide extension catheter 10' in Comparative Example 6 reached approximately the same position as the distal end of the guide extension catheter 10 in Examples 1 and 3 in the followability test into the right coronary artery 66R1, and showed better followability than the other Comparative Examples 1 to 5. However, Examples 2, and 4 to 6 showed excellent followability that can reach even more distally than Comparative Example 6.

As described above, the guide extension catheters 10 of Example 1 to 6 according to the present invention have superior followability with respect to the coronary artery 66 compared to the guide extension catheters 10' of the Comparative Examples 1 to 6 according to the conventional structure, and can be expected to reach more distally in the coronary artery 66.

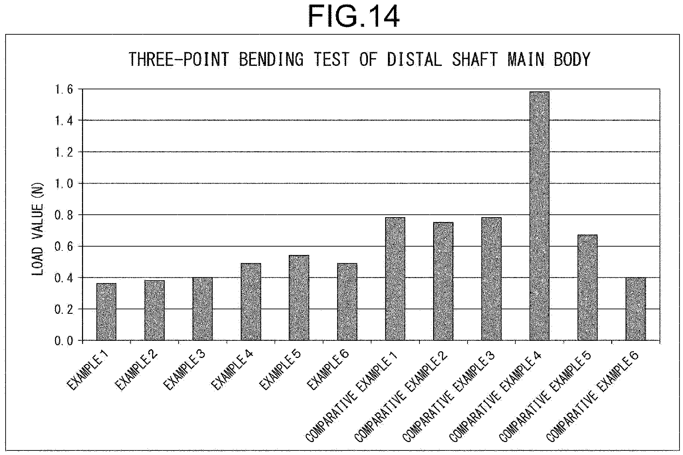

FIGS. 14 to 18 show the results of each characteristic test for the guide extension catheter 10 of Example and the guide extension catheter 10' of Comparative Example.

FIG. 14 shows the results of the three-point bending test of the distal shaft for Examples 1 to 6 and Comparative Examples 1 to 6. According to the results, the maximum load values for Examples 1 to 6 are all not greater than 0.6 N, while the maximum load values for Comparative Examples 1 to 5 are all greater than 0.6 N. The test results show that the main body 16 of the distal shaft 12 of Examples 1 to 6 is more flexible than the main body 16' of the distal shaft 12' of Comparative Examples 1 to 5, which can be bent with less force. This suggests that the main body 16 of the distal shaft 12 has excellent followability with respect to the curve of the coronary artery 66. Although the results of the three-point bending test in Comparative Example 6 were comparable to those of Examples 1 to 6, it was confirmed that the followability of Comparative Example 6 with respect to the coronary artery 66 was inferior to that of Examples 1 to 6, as described above. Thus, it became clear that good followability cannot be immediately obtained even if the distal shaft 12 is flexible.

In addition, regarding the distal shafts 12 of Examples 1 to 6, the maximum load values measured in the three-point bending test were all not smaller than 0.1 N, which means that the distal shafts 12 are hard enough to achieve the necessary pushability, etc.

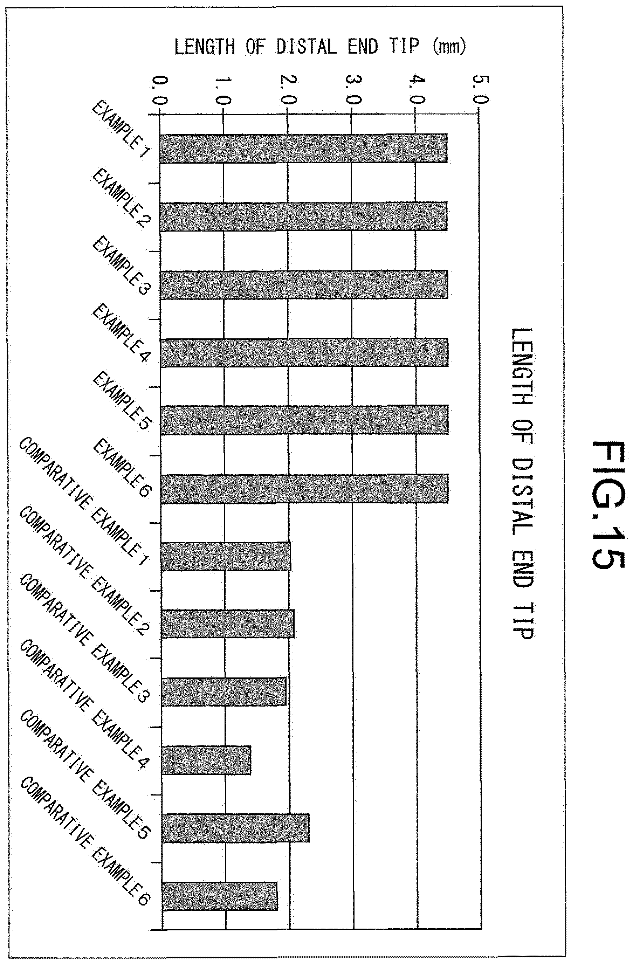

FIG. 15 shows the results of measuring the length of the distal end tip for Examples 1 to 6 and Comparative Examples 1 to 6. According to the results, the distal end tips 18 of Examples 1 to 6 are about 4.5 mm long, while the distal end tips of Comparative Examples 1 to 6 are all about 1 to 2 mm long, indicating that Examples 1 to 6 have longer distal end tips than Comparative Examples 1 to 6. Accordingly, it is conceivable that when the distal shaft 12 is inserted into the serpentine coronary artery 66, the orientation of the flexible distal end portion 26 with the long distal end tip 18 in the distal shaft 12 quickly changes to a direction along the coronary artery 66, thereby exhibiting better followability with respect to the coronary artery 66.

Example 3 and Comparative Example 6 differ in presence or absence of the ring marker, and in Comparative Example 6, the length of the distal end tip 18 is shorter than in Example 3 due to the ring marker being attached. There is a marked difference in followability of Example 3 and Comparative Example 6 with respect to the left circumflex of the left coronary artery 66L, and it has been confirmed that Example 3 has better followability than Comparative Example 6 (see FIG. 11). Accordingly, it is conceivable that one of the reasons for the improved followability of the guide extension catheter 10 with respect to the coronary artery 66 is the fact that the flexible portion is provided in the long region from the distal end in the distal end portion 26 of the distal shaft 12 by employing a flexible marker part that is formed of a resin material, which is a forming material of the distal end tip 18, mixed with a powder of radiopaque material, rather than the previously known rigid ring marker.

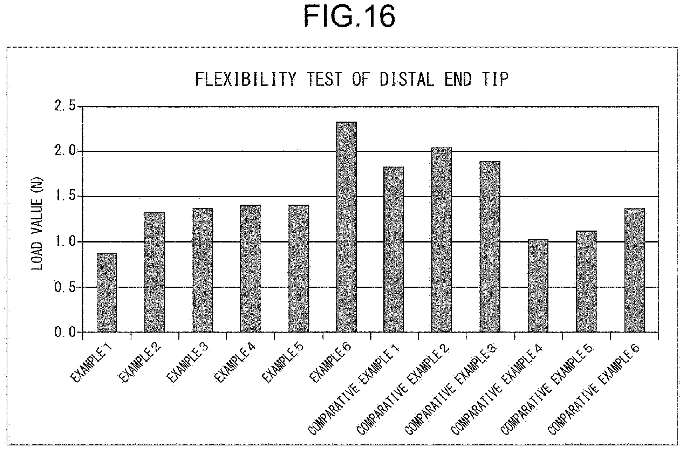

Furthermore, FIG. 16 shows the results of the flexibility test of the distal end portions of the distal shafts for Examples 1 to 6 and Comparative Examples 1 to 6. According to the results, the maximum load values for Examples 1 to 5 are all not greater than 1.5 N, while the maximum load value for Example 6 exceeds 2.0 N. Example 6 has a structure in which Example 4, which is structured following the preceding practical embodiment, includes the inner layer 20 formed of polytetrafluoroethylene (PTFE) and provided throughout the entire distal shaft 12 up to the distal end of the distal end tip 18, and the flexibility of the distal end portion is lower than in Example 4. Example 4 and Example 6 showed the same level of followability in the followability tests shown in FIGS. 10A to 12A. However, in the followability test with respect to the right coronary artery 66R2 shown in FIG. 13A, Example 4 could be inserted more distally than Example 6, and Example 4 showed better followability than Example 6.

The distal end portions 26 of the distal shafts 12 of Examples 1 to 5 are more flexible against axial compression than the distal end portions of the distal shafts 12' of Comparative Examples 1 to 3. Therefore, in Examples 1 to 5, when the distal end portion 26 of the distal shaft 12 is abutted against the wall inner surface of the coronary artery 66, the distal end portion 26 including the distal end tip 18 easily deforms preferentially to the main body 16 of the distal shaft 12, and the distal end portion 26 of the distal shaft 12 is easily oriented in a direction along the coronary artery 66. Furthermore, when the distal end portion 26 of the distal shaft 12 comes into contact with the coronary artery 66, the coronary artery 66 can be prevented from being damaged by the distal shaft 12. The maximum load value measured in the flexibility test of the distal end portion 26 of the distal shaft 12 is suitably at least 1.3 N and not greater than 1.5 N.

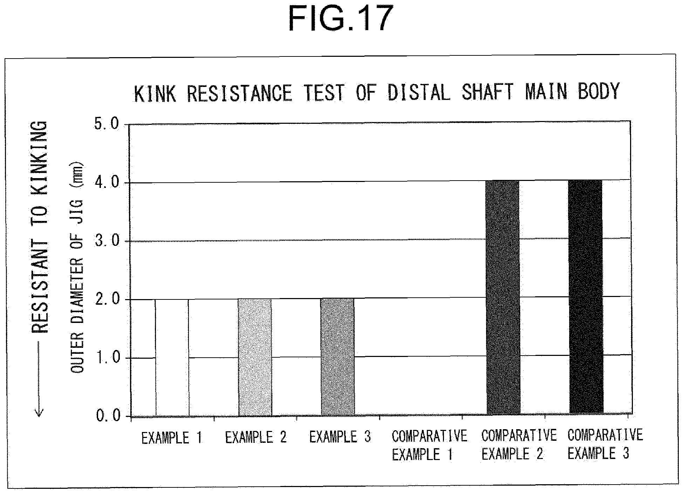

FIG. 17 shows the results of the kink resistance test of the distal shaft for Examples 1 to 3 and Comparative Examples 1 to 3. According to the results, the distal shafts 12 of Examples 1 to 3 all kinked when placed along the outer circumferential surface of the kink test jig 40 with an outer diameter of 2.0 mm, whereas the distal shafts 12' of Comparative Examples 2 and 3 kinked when placed along the outer circumferential surface of the kink test jig 40 with an outer diameter of 4.0 mm. Therefore, the distal shafts 12 of Examples 1 to 3 are resistant to kinking until they are bent with a smaller curvature compared to the distal shafts 12' of Comparative Examples 2 and 3, thereby easily avoiding adverse effects on pushability and the like due to kinking.

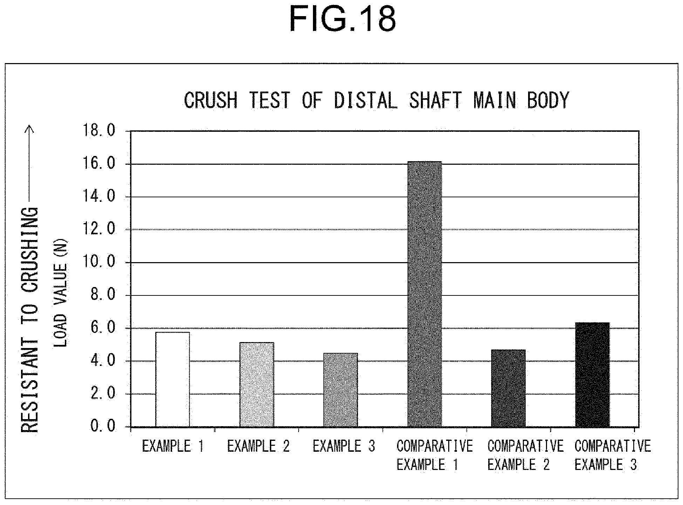

FIG. 18 shows the results of the crush tests of the distal shafts for Examples 1 to 3 and Comparative Examples 1 to 3. According to the results, the maximum load values for Examples 1 to 3 are all not smaller than 4.0 N, indicating that the shape maintenance ability in the radial direction required of the distal shaft 12 with excellent flexibility is sufficiently obtained. Besides, the maximum load values for Examples 1 to 3 are all not greater than 6.0 N, while the maximum load values for Comparative Examples 1 and 3 all exceed 6.0 N. Thus, the main bodies 16 of the distal shafts 12 of Examples 1 to 3 are more easily subjected to a change in cross-sectional shape due to the action of external force than the main bodies 16' of the distal shafts 12' of Comparative Examples 1 and 3, and are easily allowed to undergo bending deformation accompanied by a change in cross-sectional shape.

While the present invention has been described in detail hereinabove in terms of the practical embodiments, the invention is not limited by the specific description thereof. For example, in the preceding practical embodiment, the main body 16 of the distal shaft 12 has a structure in which the braided body 24 is provided between the inner layer 20 and the outer layer 22, but the main body 16 may be entirely formed of a synthetic resin material. Besides, the distal end tip 18, which is provided on the distal end side with respect to the main body 16 in the distal shaft 12, may be integrally formed with the main body 16, for example, using the same synthetic resin material as the main body 16. Alternatively, the distal end tip 18 may be made of a different material from the main body 16 and be fixed by means of welding or other means.

### KEYS TO SYMBOLS

- 10, 10': guide extension catheter
- 12, 12': distal shaft
- 14: proximal shaft
- 16, 16': main body
- 18: distal end tip (marker part)
- 20: inner layer
- 22: outer layer
- 24: braided body
- 26: distal end portion
- 28: coating layer
- 30: support base
- 32: fulcrum
- 34: indenter
- 36: holding jig
- 38: pressing jig
- 40: kink test jig
- 42: support surface
- 44: crushing jig
- 46: protecting member
- 48: connecting tube
- 50: guiding catheter
- 52: catheter main body
- 54: imaging marker
- 56: Y connector
- 58: side arm
- 60: balloon catheter
- 62: balloon
- 64: catheter assembly
- 66: coronary artery
- 68: stenosis part
- 70: femoral artery
- 72: sheath
- 74: ascending aorta
- 76: guide wire

## Claims

1. A guide extension catheter comprising:
a proximal shaft; and
a distal shaft of tubular shape provided on a distal end side of the proximal shaft, wherein
regarding a main body of the distal shaft, a maximum load value acting on the main body is at least 0.1 N and not greater than 0.6 N when the main body is placed on a support base of a test jig of a three-point bending test where a distance between fulcrums is set to 15 mm and the main body is deformed until kinking occurs by an indenter which moves closer to the support base at a relative moving speed of 10 mm/min being pressed against the main body from a lateral side at a center portion between the fulcrums of the support base,
the distal shaft includes a distal end tip that is provided on the distal end side with respect to the main body and is more flexible than the main body, and a length dimension of the distal end tip is not smaller than 2.5 mm, and
the distal end tip includes a marker part in which a powder comprising a radiopaque material is mixed.

2. The guide extension catheter according to claim 1, wherein on an outer circumferential surface of the distal shaft, a coating layer of hydrophilic polymer is provided from a distal end toward a proximal end of the distal shaft over a region having a length that does not reach the proximal end of the distal shaft.

3. The guide extension catheter according to claim 1 or 2, wherein an anti-slip part is provided on an outer circumferential surface of a proximal end portion of the distal shaft.

4. The guide extension catheter according to any one of claims 1-3, wherein when kinking occurs in the main body of the distal shaft by the main body being wound around a kink test jig having an outer circumferential surface of round tubular shape, an outer diameter dimension of the kink test jig is not greater than 3 mm.

5. The guide extension catheter according to any one of claims 1-4, wherein regarding the main body of the distal shaft, a maximum load value is not smaller than 4.0 N, the maximum load value being measured when the main body is clamped radially between a support surface and a crushing jig while being subjected to a load of 0.5 N and then the crushing jig is further moved 0.5 mm in a direction of approach to the support surface.

6. The guide extension catheter according to any one of claims 1-5, wherein regarding the distal end tip, a maximum load value is at least 0.5 N and not greater than 1.5 N, the maximum load value being measured when the distal shaft connected to the distal end tip is restrained by a holding jig at a location shifted to a proximal end side from a portion extending for 5 mm from a distal end of the distal shaft while a distal end face of the distal end tip is in contact with a pressing jig and then the holding jig is moved closer to the pressing jig at a speed of 10 mm/min to compress the distal end tip by 1 mm in a length direction.
